# EUROPEAN PATENT APPLICATION

(11) **EP 3 257 574 A1**
(43) Date of publication of application: **20.12.2017**
(21) Application number: 16174634.2
(22) Date of filing: 15.06.2016
(51) Int. Cl.: B01J 2/04, A61K 9/20

(54) **METHOD FOR PRODUCING A PARTICLE-SHAPED MATERIAL**

(71) Applicant: Clariant International Ltd, 4132 Muttenz (CH)
(72) Inventor: The designation of the inventor has not yet been filed
(74) Representative: Holmes, Rosalind

(57) **Abstract**

The invention relates to a process for producing particles from a melt, to polyethylene glycol microparticles and their use in cosmetics and/or pharmaceuticals, as a laxative or as aid in tablet production or melt granulation.

## Description

The invention relates to a process for producing particles from a melt, to polyethylene glycol microparticles and their use in cosmetics and/or pharmaceuticals, as a laxative or as aid in tablet production or melt granulation.

The selective customization of particle properties can offer intriguing opportunities for production processes and active delivery in a number of industries including pharmaceutical, healthcare, agricultural, personal care, biocide and industrial applications. The morphology of individual particles plays a central role in this pursuit, since morphology directly influences bulk powder properties, such as density, residual solvent content, and flowability.

In addition, techniques that modify particle shape and interior structure may profoundly affect product properties, such as active loading, crystallinity, release rate, solubility, and bioavailability. Thus, the ability to design particle morphology has significant implications for the production process and product attributes.

Spray drying is commonly used in the production of particles for many applications, including pharmaceuticals, food, cosmetics, fertilizers, dyes, and abrasives. Spray drying can be tailored to create a wide spectrum of particle sizes, including microparticles. Spray-dried particles are useful in a variety of biomedical and pharmaceutical applications, such as the delivery of therapeutic and diagnostic agents.

Typical applications for spray drying devices are the short-term drying of liquid solutions, suspensions (spray drying) or pasty mixtures for the rapid cooling of a melt (spray cooling or spray congealing). In general, the wet material or the melt is dispersed into the spray tower and stripped down from the spray tower to form solid particles.

Spray dryer operation influences particle characteristics. It is known in respect of drying wet material that solvent evaporation from an atomized sphere progresses through three stages: Initially, when the droplet surface is saturated with solvent, evaporation proceeds at a constant rate and is called the first stage of drying. A change in the drying rate is noted with additional drying, due to the formation of dry solids on the surface. At this critical point the surface is no longer considered to be freely saturated with solvent. Further solvent evaporation from the droplet proceeds at a slower rate, requiring diffusion or capillary action through the solid surface layer. At this stage of drying, careful operation of the spray dryer is desirable to remove as much solvent as possible and to avoid expanding the droplet and producing a low density powder. Inlet and outlet temperatures must be controlled, as well as the flow configuration of the drying gas.

It is known in respect of cooling of the melt that in a container having an interior space, a nozzle device is arranged in the upper region of the interior space. The hot melt is supplied by the nozzle device. The molten material exits the nozzle device in form of droplets or fine yarns which fall down by gravity. The feeding device for cryogenic gas is arranged in the interior space and comes into contact with these droplets of the melt. The droplets are cooled by contacting the cryogas so that its surface is no longer sticky and a powder can be obtained.

When operating a spray tower for cooling it is usual to move the gas stream through a heat exchanger in the circuit, i.e. to conduct the gas after an appropriate cooling in the spray tower. This occurs mainly in inert systems (see for example the "closed loop system" in the company brochure of the company GMF-Gouda, Waddinxveen, Netherlands). For an inert system nitrogen is highly recommended.

According to prior art, a separator unit is arranged between the spray tower and the heat exchanger for the gas flow. This separator unit is used to protect the heat exchanger from contamination. Mostly, these are cyclones as those in Schubert, H. (ed.), Handbuch der mechanischen Verfahrenstechnik, volume 2, Wiley VCH, 2002, page 883.

This technique is thus known for a long time and is generally considered to be an economic and reliable operation mode.

However, the use of such a separation unit between the spray tower and heat exchanger in the cycle gas can lead to an increased pressure loss. This reduces the cooling power, as this decreases the gas flow rate.

In the case of using a cyclone, a spiral-like flow causes a strong and sustainable change in the flow profile within the gas stream, in which defined and reproducible conditions are difficult, and also changes inside the cyclone have a direct impact on the whole process.

Furthermore, the processes for the production of spray-dried material have proved to be ineffective in their service life, since the heat exchanger had to be cleaned after relatively short periods of time because it became encrusted.

Unfortunately, the obtained microparticles exhibit an irregular shape as well as cavities. This can result in a lower bulk density.

DE 10 2004 004 968 A1 discloses a method for the production of spray-dried material in a spray tower, in which the melt fed into the tower is cooled with a counter-current of gas and then discharged from the tower while the gas stream is taken off and transferred directly to a heat exchanger to lower or raise the temperature of the gas.

However, it is often necessary for a plurality of different PEG (polyethylene glycol) powders with quite different particle size distribution to be mixed together before or during the processing. The homogeneous mixing of solids differing in particle size distribution requires great effort or is frequently just not possible with complete homogeneity.

If the fines content with particle sizes below about 100 micrometers in a PEG powder to be mixed in is too high, there is a risk of a dust explosion, additionally requiring elaborate measures such as packaging materials and systems able to eliminate electric charge, or limitation of the amounts processed each time.

The market for these applications has a high demand of powder with tailor-made grain size of certain particle shapes in large quantities at low cost as well as tailor-made bulk density.

Therefore, it is an objective of the present invention to provide a method for producing microparticles that do not exhibit the drawbacks of the prior art. In particular, the microparticles should be provided tailor-made at low costs, preferably with a high bulk density and specific particle size distribution.

The objective is solved by a process for producing particles from a melt comprising:
a) feeding a melt into at least one melt distribution device comprising a tower cone and a tower head, and generating a spray from the melt,
b) cooling the spray in the melt distribution device by using a stream of gas in a countercurrent flow to obtain a powder,
c) removing the powder obtained at the tower cone and the gas of step b) with the powder obtained at the tower head,
d) supplying the gas of step c) with the powder obtained at the tower head to a separator unit, and
e) separating the powder and the gas in the separator unit and cooling the separated gas.

Preferably, the melt distribution device comprises a tower, a tower cone and a tower head.

Preferably, the melt is a polymer melt. The melt is preferably not a suspension, emulsion or a solution. The presence of additional polymers may contribute to the final particle morphology by their interaction with the first polymer of the polymer melt.

The melt has preferably a temperature in the range from 50 to 200 °C, particularly preferred a temperature in the range from 60 to 180 °C and especially preferred in the range from 80 to 150 °C.

Specific polymers include, but are not limited to: aliphatic polyesters (e.g., poly D-lactide), sugar alcohols (e.g., sorbitol, maltitol, isomalt), carboxyalkylcelluloses (e.g., carboxymethylcellulose and crosslinked carboxymethylcellulose), alkylcelluloses (e.g., ethylcellulose), gelatins, hydroxyalkylcelluloses (e.g., hydroxymethylcellulose), hydroxyalkylalkylcelluloses (e.g., hydroxypropylmethyl cellulose), hydroxyalkylalkylcellulose derivatives (e.g. hydroxypropylmethyl cellulose acetate succinate, hydroxypropylmethyl cellulose phthalate) polyamines (e.g., chitosan), polyethylene glycols (e.g., PEG 8000, PEG 20000), methacrylic acid polymers and copolymers, homo- and copolymers of N-vinyl pyrrolidone (e.g., polyvinylpyrrolidone, crosslinked polyvinylpyrrolidone and polyvinylpyrrolidone-co-vinyl acetate), homo- and copolymers of vinyllactam, starches (e.g., cornstarch, sodium starch glycolate), polysaccharides (e.g., alginic acid), poly glycols (e.g., polypropylene glycol, polyethylene glycol), polyvinyl esters (e.g., polyvinyl acetate), shellac.

Polyethylene glycol (abbreviation PEG) is preferred as a polymer for the polymer melt. In particular preferred is polyethylene glycol with the general formula H(OCH₂CH₂)nOH, when n is above 32, corresponding to a number average molecular mass (Mn) of 1500 g/mol, solid enough to be converted into powder form. Owing to the interesting properties, it is employed in a large number of application areas. One important application area of PEGs which are solid at room temperature and in powder form with a number average molecular mass (Mn) above 1500 g/mol, preferably from 3000 to 30000 g/mol, particularly preferably from 3350 to 10000 g/mol, is the use in cosmetics and/or pharmaceuticals, as a laxative or as aid in tablet production or melt granulation. The molecular mass was determined by by calculation out of the hydroxyl value determined by titration according to DIN 53240.

The PEG can undertake very different functions depending on the process. PEG can act, for example, as lubricant and binder. Depending on the chosen molecular mass, the melting point can be so low that the PEG melts under the pressure of compression and makes so-called sinter techniques or compression techniques possible. In these cases, the PEG also acts as shaping agent and helps to maintain the tablet shape. In the specific case of melt granulation with subsequent tablet compression, the PEG can serve additionally as carrier substance, solubilizer or absorption promoter of active ingredients. In many cases, PEG with very different particle sizes is required in order to be able to fulfill simultaneously the different functions detailed above.

Blends of polymers with organic materials may also be used. The amount of the polymer in the blend may range from about 1% to about 95%, more particularly from about 5% to 90%, by weight of the blend.

The particles may also contain additional organic materials that can modify properties of the spray-cooled material. For example, certain organic substances can be included to control particle morphology/size as well as the solubility, bioavailability and release characteristics of the active ingredient. Additional organic material may also be included to further inhibit active recrystallization, further maximize active concentration and further enhance/delay/retard the dissolution rate. Additional organic materials that can be incorporated are not particularly limited. In one embodiment of this invention the additional organic material can be polymeric.

The process produces (micro)particle(s) which can also be referred to as particle-shaped material, powder, granulate, beads, spray-cooled material, fine particle or something similar. For the sake of simplicity, these (micro)particles are preferably referred in this application to powder.

Preferably, the geometry of the particles is spherical.

These kinds of powders can be used for example in injection molding techniques, production of alloys, sintering techniques, bonding materials, catalysts, paints and lacquers or cellular plastic production.

The term "melt distribution device" as used herein comprises preferably at least one device wall, at least one melt feed, at least one melt channel, at least one tower cone, at least one tower head and at least one distributor plate with one or more holes. Preferably, the distributor plate is located at the tower head.
Preferably, the melt channel, the spray cooling chamber or the spray drying chamber can also be referred to as tower, preferably spray tower.

Preferably, the melt distribution device is a spray tower or a spray dryer.

Preferably, the gas of the stream of gas in step b) has a temperature between -35 and -3 °C and the gas removed at the tower head has a temperature between -5 and 15 °C. More preferably, the gas of the stream of gas in step b) has a temperature between -35 and -3 °C and the gas removed at the tower head has a temperature between -5 and 9 °C. The temperature is measured by a PT100 platinum electrical resistance temperature sensors according to EN60751.

An additional effect on the particle morphology is the temperature profile at the solidification area near to the nozzle. A high temperature difference between melt and gas (gas outlet temperature), droplet size distribution and a high turbulence of the mixed droplets and the gas can lead to fast solidification because of high heat transfer and therefore hollow spherical particles or partly hollow particles. The effect on the quality is a reduced bulk density in comparison between hollow and full spheres. Lower gas outlet temperatures can lead to an agglomeration effect of not sufficient solidified droplets which hare still sticky and particles which are still present in the spray chamber. So for adjusting the bulk density at a certain droplet size distribution produced at the spray nozzle the solidification behavior and the agglomeration effect gives a certain particle morphology which leads to the required quality (particle size distribution, bulk density).

The temperature of the stream of gas in step b) corresponds to the inlet temperature of the gas in the melt distribution device. The temperature of the gas removed at the tower head corresponds to the outlet temperature of the gas out of the melt distribution device.

Preferably, the powder obtained at the tower cone and/or the powder obtained at the tower head are the product particles, i.e. these are the particles produced from the melt.

The spray dryer used in the process of the present invention can be any of the various commercially available apparatus. Examples of specific spray drying devices include spray dryers manufactured by Niro, Inc. (e.g., SB-Micro^{®}), the Mini Spray Dryer (Buchi Labortechnik AG), spray dryers manufactured by Spray Drying Systems, Inc. (e.g., models 30, 48, 72), and SSP Pvt. Ltd.

Spray drying processes and spray drying equipment are described generally in Perry R. H., Green D., Perry's Chemical Engineers' Handbook, Sixth Edition, McGraw-Hill Book Co., 1984, page 20-57. More details on spray drying processes and equipment are reviewed by Marshall W. R., 'Atomization and Spray Drying', Chem. Eng. Prog. Monogr. Series 2, 50, 1954. The contents of these references are hereby incorporated by reference and can be used for spray cooling as well.

Preferably, the temperature and flow rate of the gas stream (drying gas or cooling gas) and the design of the spray dryer (melt distribution device) are chosen so that the atomized droplets are dry enough by the time they reach the wall of the tower that they are essentially solid and so that they form a powder and do not stick to the wall. The actual length of time to achieve this level of dryness depends on the size of the droplets of the spray, the formulation, and spray dryer. Following the solidification, the (solid) powder may stay in the spray drying chamber (tower) for 5 - 60 seconds.

In step a) according the present invention a melt is fed into at least one melt distribution device comprising a tower cone and a tower head, and a spray is generated from the melt. Feeding and supplying can be used in this context simultaneously. Preferably, the melt is fed into a distributor plate, preferably a drop-forming nozzle, and is converted into droplets forming a spray which afterwards can be solidified to powder (particles). The droplet size can fix the later (particle) size of the powder. The droplet size depends on the one hand on the pressure with which the melt is forced through the drop-forming nozzle, and on the other hand on the nozzle geometry.

A further consequence of this engineered particle morphology in accordance with certain aspects of the invention is the increase in bulk powder density. Increased powder density is an important attribute for many applications, including pharmaceutics, health care, personal care, agriculture, biocide, and industrial chemicals. When the melt comprises a polymer, the extent of its collapse - and therefore the net effect on the spray-cooled powder properties - depends on polymer factors, such as the polymer chemical structure and molecular weight, the streaming rate of the gas (nitrogen) and the size of the droplet leaving the nozzle.

The melt can be introduced in the tower at a temperature between 60 and 140 °C, preferably between 70 and 120 °C, particularly preferably between 80 and 110 °C. The temperatures of the heated nozzles are likewise within these ranges. The nozzle orifice diameters can be from 0.05 to 5 mm, preferably 0.1 to 3 mm, particularly preferably 0.5 to 2 mm.

The pressure with which the melt is forced through the nozzle device depends frequently on the viscosity. For polymers this is dependent on the average chain length, i.e. the (number) average molecular mass of the PEG (polyethylene glycol) to be solidified, and on the temperature used.
The pressure typically can be used is from 0.1 to 100 bar, preferably from 2 to 45 bar, particularly preferably from 5 to 70 bar.

The droplets can be solidified to (powder) particles by using all conventional coolants such as, for example, air or cryogases such as nitrogen or carbon dioxide.

Production is possible from the melt, preferably a polyethylene glycol melt, by conventional processes for spraying and cooling melts, for example by the process described in EP 0 945 173, or by conventional processes for dropletizing and cooling melts with vibrating nozzle orifice plates.

In step b) according the present invention the spray is cooled in the melt distribution device by using a stream of gas in a countercurrent flow to the spray to obtain a powder. Preferably, the spray falls down by gravity. Preferably, the melt distribution device is a spray dryer. Preferably, the spray dryer comprises a tower, a tower cone and a tower head. Particularly preferred, the tower cone and the tower head are arranged at opposite ends of the tower. The tower can be tube-shaped.

Preferably, in step b) the spray is cooled. In other words, in step b) the spray generated from the melt is spray-cooled. The terms "spray drying" and "spray cooling" are used conventionally and, preferably, refer to processes involving breaking up liquid mixtures into (small) droplets in a container (spray dryer), preferably followed by drying. Atomization techniques to form droplets include for example pressure nozzles or rotary atomizers.

Preferably, single-substance nozzles are located at the tower head of the spray dryer. Preferably, the melt is brought into rotation, whereby a hollow cone of the melt is formed at the nozzle orifice. Preferably, this hollow cone disintegrates the melt into droplets and a spray is formed. Preferably, a gas stream is formed at the tower cone and streams in a countercurrent flow to the spray formed by the former melt. Preferably, the spray is cooled to powder. Advantageously, no powder sticks to the wall of the melt distribution device, with other words to the inner walls of the spray dryer.

Preferably, the stream of gas contains nitrogen, preferably at least 5% by weight of nitrogen, based on the total weight of the stream of gas, more preferably 50% to 99% by weight of nitrogen, based on the total weight of the stream of gas. Particularly preferred, the gas of the stream of gas consists of nitrogen.

Preferably, the stream of gas in step b) has a temperature between -45 and 30 °C, more preferably between -30 and -10 °C. Preferably, the gas removed at the tower head has a temperature between -5 and 9 °C, more preferably between -3 and 4 °C. The temperature is measured by a PT100 platinum electrical resistance temperature sensors according to EN60751.

Preferably, the stream of gas in step b) has a specific gas flow rate, from 600 to 1500 m/h, more preferably from 763 to 1273 m/h.

In step c) according the present invention the powder obtained at the tower cone and the gas of step b) with the powder obtained at the tower head are removed. This means that the powder obtained at the tower cone is removed and the gas of step b) is removed together with the powder obtained at the tower head. Preferably, the powder is removed at the tower cone via a rotary valve. Preferably, the gas with the powder is removed via at least one outlet connections.

In step d) according the present invention the gas of step c) with the powder is supplied to a separator unit. Preferably, the separator unit is a cyclone.

In step e) according the present invention the powder and the gas in the separator unit are separated and the separated gas is cooled afterwards. The gas separated can be filtered to remove smaller particles. Preferably, the gas purified by the filter contains only (fine) particles up to 20 mg/m³. Preferably, the filter is a fully-automated bag filter.

Preferably, the powder from step e) is mixed with the powder obtained at the tower cone.

Preferably, after the gas has been purified by a filter it can be cooled and released to the atmosphere or it can be used again in step a).

Preferably, the inventive process comprises the steps:
a) feeding a polyethylene glycol (PEG) melt into at least one spray dryer comprising a spray tower, whereby the pressure in the spray tower is in the range from 10 to 90 mbar, the temperature in the spray tower is in the range from -30 to 10 °C, wherein the PEG melt is distributed in spray nozzles by jet break-up at a pressure nozzle into spray at a pressure in the range from 10 to 200 bar, at a temperature in the range from 110 to 140 °C, and at a specific volume-based throughput of solids in the range from 3.07 to 6.6 kg/(m³ volume of spray tower·h),
b) cooling the PEG spray in the spray dryer by using a gas stream of nitrogen in a countercurrent flow to obtain a powder, wherein nitrogen is fed in at the bottom part of the spray tower having a pressure in the range from 10 to 90 mbar, a temperature in the range from -30 to -2.5 °C, preferably from -30 to -10 °C, and a superficial velocity in the spray tower from 763 to 1273 m/h via a gas distributor ring,
c) removing the powder obtained at the tower cone and the nitrogen of step b) with the powder obtained at the tower head via a gas outlet at the spray tower head, wherein the powder obtained at the tower cone is removed via a rotary valve,
d) supplying the nitrogen of step c) with the powder to a cyclone, for separation of particles being present in the gas,
e) separating the powder and the nitrogen in the cyclone, wherein the nitrogen which is withdrawn from the cyclone is fed into a continuous back filter with filter bags for separation of fine dust, wherein the residual content of dust is lowered to less than 20 mg/m³, the filter bags are purged during operation by pressure gas pulses and fine dust in the filter is removed from a dust collecting pan which is located below the filter, and wherein the separated nitrogen is cooled in a cooling circuit in a gas circulation cooler and recycled into the spray tower, and
f) the powder being removed in the cyclone is mixed with the powder removed via the rotary valve and sieved in a sieve having a mesh size of 2 mm to separate oversized particles.

Preferably, the powder, in particular the powder at the tower cone, has an average particle size distribution, whereby

| | |
|---|---|
| B1.1 | 18 to 32 wt % has a particle size of less than 90 µm, |
| B1.2 | 45 to 65 wt % has a particles size in the range from 90 to 200 µm, |
| B1.3 | 13 to 26 wt % has a particle size in the range from 200 to 400 µm and |
| B1.4 | the mean particle diameter d50 is 100 to 140 µm, preferably 115 to 125 µm, |

or

| | |
|---|---|
| B2.1 | 4 to 12 wt % has a particles size of less than 90 µm, |
| B2.2 | 25 to 42 wt % has a particle size in the range from 90 to 200 µm, |
| B2.3 | 40 to 57 wt % has a particle size in the range from 200 to 400 µm, |
| B2.4 | 5 to 17 wt % has a particle size in the range from 400 to 630 µm and |
| B2.5 | the mean particle diameter d50 is 200 to 240 µm, preferably 215 to 225 µm, |

or

| | |
|---|---|
| B3.1 | less than 5 wt % has a particle size of less than 90 µm, |
| B3.2 | 12 to 23 wt % has a particle size in the range from 90 to 200 µm, |
| B3.3 | 40 to 60 wt % has a particle size in the range from 200 to 400 µm, |
| B3.4 | 20 to 0 wt % has a particle size in the range from 400 to 630 µm and |
| B3.5 | the mean particle diameter d50 is 260 to 300 µm, preferably 275 to 285 µm |

This particle size and the particle size distribution can be determined via state of the art analysis techniques by laser diffraction, e.g. via analysis apparatus like Mastersizer 2000 or 3000 by Malvern Instruments GmbH. The particle size distribution in the sense of the invention is characterized by the D10-, D50-, and D-90 value. The definition of D10 is: that equivalent diameter where 10 mass % (of the particles) of the sample has a smaller diameter and hence the remaining 90% is coarser. The definition of D50 and D90 can be derived similarly (see: HORIBA Scientific, A Guidebook to Particle Size Analysis, 2014, page 6).

Preferably, the powder obtained at the tower cone and/or the powder obtained at the tower cone mixed with powder from step e) has a bulk density in the range from 500 to 700 kg/m³, more preferably from 650 to 730 kg/m³.

In a further embodiment of the invention polyethylene glycol microparticles obtained by
(a) feeding a polyethylene glycol melt into at least one melt distribution device comprising a tower cone and a tower head, and generating a spray from the melt,
(b) cooling the polyethylene glycol spray in a melt distribution device by using a stream of gas in a countercurrent flow to obtain polyethylene glycol microparticles,
(c) removing the polyethylene glycol microparticles obtained at the tower cone and the gas of step (b) with the polyethylene glycol microparticles obtained at the tower head,
(d) supplying the gas of step (c) with the polyethylene glycol microparticles to a separator unit, and
(e) separating the polyethylene glycol microparticles and the gas in the separator unit and cooling the separated gas.

Preferably, the polyethylene glycol microparticles from step (e) are mixed with the polyethylene glycol microparticles obtained at the tower cone.
The polyethylene glycol microparticles glycol microparticles preferably have an average particle size distribution, whereby

| | |
|---|---|
| B1.1 | 18 to 32 wt % have a particle size of less than 90 µm, |
| B1.2 | 45 to 65 wt % have a particles size in the range from 90 to 200 µm, |
| B1.3 | 13 to 26 wt % have a particle size in the range from 200 to 400 µm and |
| B1.4 | the mean particle diameter d50 is 100 to 140 µm, preferably 115 to 125 µm, |

or

| | |
|---|---|
| B2.1 | 4 to 12 wt % have a particles size of less than 90 µm, |
| B2.2 | 25 to 42 wt % have a particle size in the range from 90 to 200 µm, |
| B2.3 | 40 to 57 wt % have a particle size in the range from 200 to 400 µm, |
| B2.4 | 5 to 17 wt % have a particle size in the range from 400 to 630 µm and |
| B2.5 | the mean particle diameter d50 is 200 to 240 µm, preferably 215 to 225 µm, |

or

| | |
|---|---|
| B3.1 | less than 5 wt % have a particle size of less than 90 µm, |
| B3.2 | 12 to 23 wt % have a particle size in the range from 90 to 200 µm, |
| B3.3 | 40 to 60 wt % have a particle size in the range from 200 to 400 µm, |
| B3.4 | 20 to 0 wt % have a particle size in the range from 400 to 630 µm and |
| B3.5 | the mean particle diameter d50 is 260 to 300 µm, preferably 275 to 285 µm |

A further embodiment of the invention is the use of the inventive polyethylene glycol microparticles in cosmetics and/or pharmaceuticals.

A further embodiment of the invention is the use of the inventive polyethylene glycol microparticles as a laxative.

A further embodiment of the invention is the use of the inventive polyethylene glycol microparticles as aid in tablet production and/or melt granulation.

Microparticles of the invention may be presented in numerous forms commonly used in a wide variety of industries. Exemplary presentation forms are powders, granules, and multiparticulates. These forms may be used directly or further processed to produce tablets, capsules, or pills, or reconstituted by addition of water or other liquids to form a paste, slurry, suspension or solution. Various additives may be mixed, ground, or granulated with the microparticles of this invention to form a material suitable for the above product forms.
All embodiments, preferred embodiments and subjects beginning with "preferably" or "more preferably" listed above can be combined freely with one another, unless it is clear from the context that this is not possible.

The term "comprising" encompasses the more restrictive terms "consisting essentially of' and "consisting of".

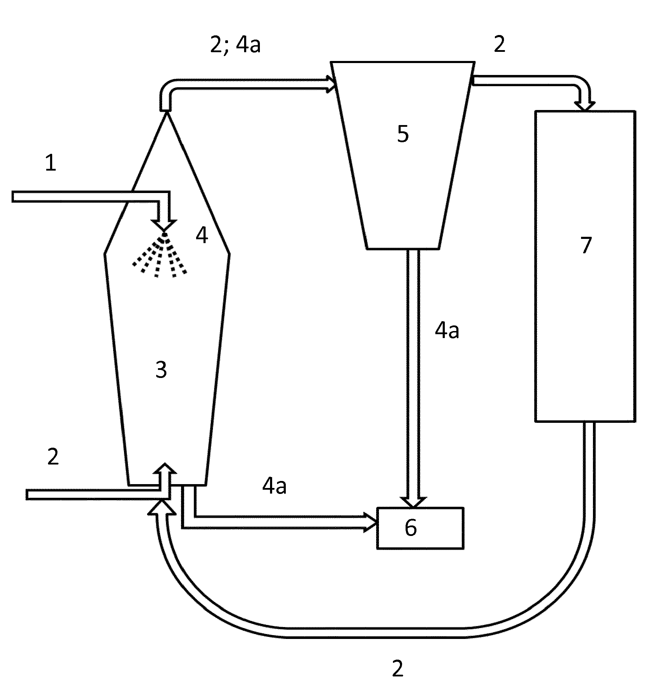

The invention will be illustrated in figure 1.

Figure 1 illustrates schematically the process according to the present invention.

### Reference signs:

- 1:: melt, especially polyethylene glycol melt
- 2:: stream of gas, especially nitrogen
- 3:: melt distribution device, especially spray dryer
- 4:: spray
- 4a:: powder
- 5:: separator unit, especially cyclone
- 6:: mixing unit
- 7:: filter, especially continuous back filter

In Figure 1, the polyethylene glycol (PEG) melt (1) is supplied via a line to the spray dryer (3) at the tower head. Additionally, nitrogen (2) is supplied via a further line to the tower cone of the spray dryer (3). The molten polyethylene glycol (1) exits the nozzle (not illustrated) in the spray dryer (3) in form of a spray (4). The spray (4) falls down by gravity. The nitrogen (2) supplied via a line streams in a countercurrent flow to the spray (4). Thereby, a powder (4a) is obtained (not shown). Via a line, nitrogen (2) and powder (4a) of the polyethylene glycol is removed and supplied to the cyclone (5). In the cyclone (5), the powder (4a) and the nitrogen (2) are separated. The powder (4a) leaves the cyclone (5) via a line to a mixing unit (6). The nitrogen (2) is removed via a line to a continuous back filter (7). The back filter (7) separates particles (fine dust of polyethylene glycol) still present in the nitrogen. The purified nitrogen (2) is afterwards supplied to the spray dryer (3) via a line. The line coming from the back filter (7) is connected to the line in which nitrogen (2) is supplied to the tower cone. Alternatively, a line coming from the back filter (7) can be connected to the spray dryer (3) directly (not illustrated). The powder (4a) obtained at the tower cone of the spray dryer (3) is supplied via a line in a mixing unit (6) and mixed with the powder (4a) obtained in the cyclone (5) which is supplied to the mixing unit (6) via a line.

The invention is elucidated in more detail by the examples hereinafter.

### 1 Raw material

Polyglycol 3350 and Polyglycol 4000.

### 1.1 Drop point / viscosity

The viscosity of the PEG 3350 melt is 70 to 75 mPas at 120 °C. The drop point is at 80 °C.
PEG 3350 Op 01/11 sample 1 = 71.76 mPas at 120 °C
PEG 3350 Op 05/11 sample 15 = 74.37 mPas at 120 °C
PEG 3350 Op07/11 sample 2 = 72.15 mPas at 120 °C

### 2 Process principle

For the spray congealing process in the modified spray dryer pilot plant Frankfurt the modified spray drying equipment is used. The spray tower is operated in counter-current mode. That means by modification of the tower head and the tower cone the tower is operated by an inversion of the gas stream. So instead of co-current spray drying the process operates in counter-current mode.

The plant uses single-substance hollow cone pressure nozzles as atomising device. Due to the modification of the tower head, the nitrogen gas flow leaves the tower at the tower head. As a result of the installation of a modified gas inlet at the tower cone, the feeding of cold gas at the tower cone is possible.

The PEG melt is pumped from the feeding vessel through high pressure heat jacketed pipes towards the tower head and sprayed by a hollow cone pressure nozzle at the tower head. In the pressure nozzle the liquid melt is accelerated and rotates before reaching the nozzle orifice. By this rotation the liquid leaves the nozzle in a hollow cone spray with defined angle. The atomizing takes place by the disaggregation of the melt jet stream which leaves the nozzle orifice and forms a defined droplet size distribution of the spray. The energy supply for the atomization of the melt primarily results from the energy of pressure increase.

The cooling nitrogen gas enters the spray tower by the filling valve from the N₂ piping at 2.5 bar. The nitrogen flow is reduced to 30 mbar over pressure and cooled down in a in a heat exchanger. The gas flow enters the tower at a first floor at the tower cone in counter-current mode to the dust (spray) of the liquid droplets and is evenly conducted from the bottom to the top of the spray tower by means.

In the spray tower the atomized melt droplets and the cooling gas are mixed intensively. Due to the enormous surface of the sprayed droplets and the cold gas the heat transfer is very high so that the spray solidifies in a sufficient velocity, before the spray can reach the wall and deposit at the tower wall.

Below the tower cone at the bottom the solidified coarse material (of the polymer particles) is discharged by a rotary valve. The gas loaded with fine particles (of the polymer particles) funnels via a gas withdrawal at the tower head laterally es to the subsequent separator unit.

The separator unit downstream of the spray tower consists of a Cyclone, wherein the fine particles are extensively removed from the cooling gas and discharged via a hand a rotary valve.

Due to the formed droplet size distribution at the nozzle, the particle properties and the gas velocity in the tower the load of fine particles towards the cyclone varies from 1 % to 5%. Depending on the particle properties, the residual dust content of the gas flow discharged from the cyclone is much higher than the permissible values for dust content in exhaust air. Therefore, the cooling gas is funneled from the cyclone to a filter, where the fine particles are separated and the residual dust content is decreased to values lower than 20 mg/m³.

The filter is a continuous, fully automatic bag filter, whose filter hoses are purged by pressure gas pulses during operation.

The fine dust in the filter is removed by means of a hand valve from a dust collecting container, which is located below the filter.

The cooling gas enters a fan (Ventilator V0673) and leaves the plant by the roof. In the production scale the gas will be recycled to the circulation gas cooler and will be reintroduced into the spray tower.

The tower product (polymer particles), which is discharged by a rotary valve, is continuously mixed with the discharged fine particles (of the polymer particles) from the cyclone, sieved to avoid oversized product and packed. The material which is separated over the filter is waste.

Single components used for product separation can be added to or removed from the gas path as well as the product path.
In counter-current mode the melt flow is injected at the tower head against the direction of the gas flow. The product falls in the opposite direction of the ascending cooling gas and is separated at the tower cone

### 2.1 Description of plant

The boundary conditions for the production tower are a maximum cylindrical height of >6 m because of the maximum height of building, a cylindrical height of >4 m and a certain diameter. The mass flow rate of powder material is 50 kg/h. The productivity is calculated within 3 kg/hm³.

### 2.2 Cooling capacity

The required cooling capacity for congealing of the PEG melt (kg melt/h) is dependent from the inlet and the outlet temperature as well as the cooling gas flow rate.

### 3 Experimental procedure

### 3.1 Production of spray melt

PEG 3350 (average molecular weight of 2931 to 3769 g/mol; trademark "CARBOWAX™ Polyethylene Glycol (PEG) 3350" of The Dow Chemical Company) is submitted to the feeding vessel in the form of granules, prills or powder and heated by jacket with steam.

During spray drying the melts are continuously supplied to the nozzle in the spray tower via a high pressure pump. The pipes leading to the nozzle tower are heated by jacketed pipes with steam.

### 3.2 Spray drying / Spray congealing

In the course of a pilot run the process parameters and the quality of atomization are adapted to the viscosity of the melt and temperatures as well as the minimal required mass and volume flows are examined.

At the beginning a hollow cone pressure nozzle with 0.6 mm orifice diameter with a defined spray angle made of stainless steel, W.-Nr. 1.4571, is used. Hereby, the highest possible coverage of the cone at a maximal possible retention time of the drops until they reach the walls of the dryer is achieved. The occurrence of wall covering is not observed on any account.

In further experiments further pressure nozzles with a diameter of 0.4 to 0.7 mm made of stainless steel, W.-Nr. 1.4571, are used.

Care is taken that temperature level and temperature difference of the cooling gas enable a constant operation of the plant. Further, it is ensured that a sufficient distance to the limit temperatures is kept and additionally the variation of temperatures by changes in the melting process is possible.

The gas flow velocity is not higher than 0.3 m/s because of particle discharge into the exhaust gas. So the minimum gas inlet temperature into the tower is -30 °C. Maximum gas outlet is 6 °C. The nitrogen cooling gas is circulated in a closed loop over a heat exchanger.

The maximal atomization temperatures are varied from 80 to 200 °C for each product. The cooling gas temperature is varied from -30 to 6 °C depending on product and achievable particle quality.

Moreover, the inlet and outlet temperature as well as the thereto via control technology coupled power of the feed pump are varied. Further, the pressure in front of the nozzle is varied by changing the throughput of the melt or the nozzle size, respectively.

The dust explosion ability can be disregarded in case of inerting with nitrogen. Thermal decomposition is determined by DTA and DSC measurements in order to determine the maximal melting temperature at a maximal reduction of the viscosity before atomization.

4 Results of spray experiments to achieve particles having a PEG 3350 specification in counter-current mode

In all experiments spherical PEG particles are produced. Gas inclusions or hollow spheres are observed depending on temperature profile and procedure.

Table 1 shows the required particle size distribution and the required span of the particle distribution has a major effect on the bulk density.

From theory monomodal distributed spherical particles at most dense sphere packing have a porosity of 0.3. So to adjust the bulk density in the required range, additional parameters effect it. The morphology of the particles hollow spheres, particles with partly gas entrainments or full dense spheres and surface properties have an impact on the bulk density of the particles at different particles size distributions.

With adequate process parameters as mentioned and shown in several pilot trials, see table 1, adequate temperature profiles and flow characteristics, the morphology of the particles and the bulk density at different particle size distribution can be adjusted accordingly to the required specification as shown in table 1.

**Table 1**

| Trial | OP5/12 | OP5/12 | OP5/12 | OP5/12 | OP18/12 | OP16/12 | OP17/12 | OP17/12 | OP32/12 | OP34/12 | OP7/13 |
|---|---|---|---|---|---|---|---|---|---|---|---|
| Sampling time | 30 bar Average sample | 60 bar Average sample | 30 bar Composite sample | 60 bar Composite sample | 11.15 a.m. | 12.30 a.m. | 12.55 a.m. | 3 p.m. | average | average | average |
| Particle size < 90 µm [I] [%] | 6.4 | 17.2 | 10.4 | 20.3 | 10.1 | 19.8 | 9.8 | 13.3 | 1,838 | 2,245 | 2,6 |
| Particle size 90 - 200 µm [I] [%] | 31.6 | 58.0 | 40.2 | 60.1 | 34.9 | 55.7 | 35.0 | 55.0 | 23,506 | 17,920 | 21,3 |
| Particle size 200 - 400 µm [I] [%] | 43.0 | 23.7 | 43.0 | 19.4 | 46.7 | 23.7 | 47.1 | 30.4 | 56,001 | 58,047 | 61,4 |
| Particle size 400 - 630 µm [I] [%] | 17.2 | 1.0 | 6.4 | 0.2 | 8.2 | 0.7 | 8.1 | 1.3 | 15,927 | 18,851 | 14,6 |
| Particle size > 400 µm [I] [%] | 18.9 | 1.0 | 6.4 | 0.2 | 8.2 | 0.7 | 8.1 | 1.3 | 2,083 | 2,183 | 1,6 |
| Particle size > 630 µm [I] [%] | 0.8 | 0.0 | 0.0 | 0.0 | 0.0 | 0.0 | 0.0 | 0.0 | 1,838 | 0,755 | 0,29 |
| Particle size > 680 µm (protective strainer) [I] [%] | 0.1 | 0.0 | 0.0 | 0.0 | 0.0 | 0.0 | 0.0 | 0.0 | 0.0 | 0 | 0 |
| Particle size > 800 Lim (control sieve) [I] [%] | 0.0 | 0.0 | 0.0 | 0.0 | 0.0 | 0.0 | 0.0 | 0.0 | 0.0 | 0 | 0 |
| Bulk density - loose [g/L] | 656 | 587 | 659 | 587 | 653 | 605 | 644 | 596 | 619 | 713 | 665 |

Deposition of not congealed PEG-droplets in the area of the spray tower is not observed. The walls of the spray tower are powdered with fine particles.

Due to the strongly accelerated gas flow in the immediate vicinity of the nozzle, large gas volumes are pulled in the spray cone from the surrounding area. This leads to circulation of gas in the area of the spray nozzle, which pulls single, already congealed PEG particles in the spray cone of the PEG melt. In this process, a granulation effect is observed, which leads to particles having much larger particle diameters than the maximal produced droplet size.

### 5 Comparison examples (prior art) vs. inventive example

The process disclosed in DE 10 2004 004 968 was carried out on a pilot plant. As it can be seen in table 2 of the present application, the process disclosed in DE 10 2004 004 968 leads to a yield in the range to 96%. The bulk density is in the range from 630 to 640 kg/m³.

In the first and in the second column of table 2 of the present application, the comparative example and the inventive example of the DE 10 2004 004 968 are illustrated. As this document does not describe any yield, the inventor has carried this process on a pilot plant (see third and fourth column in table 2). The third column corresponds to the comparative example in table 2 and the fourth column to the inventive example of table 2.

Due to the strongly accelerated gas flow in the immediate vicinity of the nozzle, large gas volumes are pulled in the spray cone from the surrounding area. This leads to circulation of gas in the area of the spray nozzle, which pulls single, already congealed PEG particles in the spray cone of the PEG melt. In this process, an unwanted granulation effect is observed, which leads to particles having much larger particle diameters than the maximal produced droplet size. An increasing outlet temperature leads to higher agglomeration effect which has a negative effect on the bulk density and particle size distribution (psd). The bulk density drops with increasing agglomeration of particles. So bulk density > 640 kg/m³ is not achieved anymore.

This particle size and the particle size distribution can be determined via state of the art analysis techniques by laser diffraction, e.g. via analysis apparatus like Mastersizer 2000 or 3000 by Malvern Instruments GmbH. The particle size distribution in the sense of the invention is characterized by the D10-, D50-, and D-90 value. The definition of D10 is: that equivalent diameter where 10 mass % (of the particles) of the sample has a smaller diameter and hence the remaining 90% is coarser. The definition of D50 and D90 can be derived similarly (see: HORIBA Scientific, A Guidebook to Particle Size Analysis, 2014, page 6).

All observations during the trials at outlet temperatures > 10 °C had a strong negative influence on the particle size distribution (psd) and the bulk density and the yield. Therefore at outlet temperatures > 10 °C no further investigation was done because it will not lead to the required quality and operating performance.

To reduce the agglomeration, adjusting the required particle morphology and to observe the high quality product the outlet temperature was limited to max 10 °C in all pilot trials. By increasing the outlet temperature the yield is lower due to not sufficient solidified particles which leave the tower and deposit on the wall of the pipes and the cyclone.

**Table 2**

| | Comparative example in DE102004004968 | Inventive example in DE102004004968 | Clariant Pilot plant according to DE102004004968 | Clariant Pilot plant according to DE102004004968 |
|---|---|---|---|---|
| process flow | - | Counter current | Counter current | Counter current |
| inlet temp [°C] | -5 | -5 | -5 | -3 |
| outlet temp [°C] | 24 | 18 | 1 | 10 |
| yield [%] | <96 | < 96 | 96 | 96 |
| bulk density [kg/m³] | - | - | 640 | 630 |
| d50 [µm] | - | - | 260 | 220 |

In table 3 the results for the process of the present invention are disclosed. The yield is higher as in the process carried out on the pilot plant for the DE 10 2004 004 968. Even more, the bulk density is higher. Even more, if d50 is in the range 230 to 240 µm, the bulk density is higher.

**Table 3**

| | Clariant Pilot plant | Clariant Pilot plant |
|---|---|---|
| process flow | Counter current | Counter current |
| inlet temp [°C] | -28 | -15 |
| outlet temp [°C] | 5 | 1 |
| yield [%] | 99 | 99 |
| bulk density [kg/m³] | 670 | 670 |
| d50 [µm] | 230-240 | 300 |

## Claims

1. A process for producing particles from a melt comprising:
a) feeding a melt into at least one melt distribution device comprising a tower cone and a tower head, and generating a spray from the melt,
b) cooling the spray in the melt distribution device by using a stream of gas in a countercurrent flow to obtain a powder,
c) removing the powder obtained at the tower cone and the gas of step b) with the powder obtained at the tower head,
d) supplying the gas of step c) with the powder obtained at the tower head to a separator unit, and
e) separating the powder and the gas in the separator unit and cooling the separated gas.

2. The process according to claim 1, wherein the gas of the stream of gas in step b) has a temperature between -35 and -3 °C and the gas removed at the tower head has a temperature between -5 and 15 °C.

3. The process according to claim 1 or claim 2, wherein the powder obtained at the tower cone and/or the powder obtained at the tower head are the product particles.

4. The process according to any of the claims 1 to 3, wherein the melt distribution device is a spray dryer.

5. The process according to any of the claims 1 to 4, wherein separator unit is a cyclone.

6. The process according to any of the claims 1 to 5, wherein the melt is a polymer melt.

7. The process according to any of the claims 1 to 6, wherein the stream of gas contains nitrogen.

8. The process according to any of the claims 1 to 7, wherein the stream of gas in step b) has a temperature between -45 and 30 °C.

9. The process according to any of the claims 1 to 8, whereby the powder at the tower cone has an average particle size distribution, whereby
B1.1 18 to 32 wt % has a particle size of less than 90 µm,
B1.2 45 to 65 wt % has a particles size in the range from 90 to 200 µm,
B1.3 13 to 26 wt % has a particle size in the range from 200 to 400 µm and
B1.4 the mean particle diameter d50 is 100 to 140 µm
or
B2.1 4 to 12 wt % has a particles size of less than 90 µm,
B2.2 25 to 42 wt % has a particle size in the range from 90 to 200 µm,
B2.3 40 to 57 wt % has a particle size in the range from 200 to 400 µm,
B2.4 5 to 17 wt % has a particle size in the range from 400 to 630 µm and
B2.5 the mean particle diameter d50 is 200 to 240 µm
or
B3.1 less than 5 wt % has a particle size of less than 90 µm,
B3.2 12 to 23 wt % has a particle size in the range from 90 to 200 µm,
B3.3 40 to 60 wt % has a particle size in the range from 200 to 400 µm,
B3.4 20 to 0 wt % has a particle size in the range from 400 to 630 µm and
B3.5 the mean particle diameter d50 is 260 to 300 µm.

10. The process according to any of the claims 1 to 9, wherein the powder from step e) is mixed with the powder obtained at the tower cone.

11. The process according to any of the claims 1 to 10, wherein the powder obtained at the tower cone and/or the powder obtained at the tower cone mixed with powder from step e) has a bulk density in the range from 500 to 700 kg/m³.

12. Polyethylene glycol microparticles obtained by
(a) feeding a polyethylene glycol melt into at least one melt distribution device comprising a tower cone and a tower head, and generating a spray from the melt,
(b) cooling the polyethylene glycol spray in a melt distribution device by using a stream of gas in a countercurrent flow to obtain polyethylene glycol microparticles,
(c) removing the polyethylene glycol microparticles obtained at the tower cone and the gas of step (b) with the polyethylene glycol microparticles obtained at the tower head,
(d) supplying the gas of step (c) with the polyethylene glycol microparticles to a separator unit, and
(e) separating the polyethylene glycol microparticles and the gas in the separator unit and cooling the separated gas.

13. The polyethylene glycol microparticles according to claim 12, wherein the polyethylene glycol microparticles have an average particle size distribution, whereby
B1.1 18 to 32 wt % have a particle size of less than 90 µm,
B1.2 45 to 65 wt % have a particles size in the range from 90 to 200 µm,
B1.3 13 to 26 wt % have a particle size in the range from 200 to 400 µm and
B1.4 the mean particle diameter d50 is 100 to 140 µm
or
B2.1 4 to 12 wt % have a particles size of less than 90 µm,
B2.2 25 to 42 wt % have a particle size in the range from 90 to 200 µm,
B2.3 40 to 57 wt % have a particle size in the range from 200 to 400 µm,
B2.4 5 to 17 wt % have a particle size in the range from 400 to 630 µm and
B2.5 the mean particle diameter d50 is 200 to 240 µm
or
B3.1 less than 5 wt % have a particle size of less than 90 µm,
B3.2 12 to 23 wt % have a particle size in the range from 90 to 200 µm,
B3.3 40 to 60 wt % have a particle size in the range from 200 to 400 µm,
B3.4 20 to 0 wt % have a particle size in the range from 400 to 630 µm and
B3.5 the mean particle diameter d50 is 260 to 300 µm.

14. Use of the polyethylene glycol microparticles according to claims 12 or 13 in cosmetics and/or pharmaceuticals.

15. Use of the polyethylene glycol microparticles according to claims 12 or 13 as a laxative.

16. Use of the polyethylene glycol microparticles according to claims 12 or 13 as aid in tablet production or melt granulation.
